# FASCICULE DE BREVET EUROPEEN

(11) **EP 2 935 426 B1**
(45) Date de publication et mention de la délivrance du brevet: **12.08.2020**
(21) Numéro de dépôt: 13818321.5
(22) Date de dépôt: 17.12.2013
(51) Int. Cl.: C08J 9/28, A61L 15/44, B29C 44/12, A61F 13/00

(54) **PROCEDE D'INCORPORATION D'AGENTS ACTIFS AU SEIN D'UNE MOUSSE POLYMERE HYDROPHILE**
VERFAHREN ZUM EINBRINGEN VON WIRKSTOFFEN IN EINEN HYDROPHILEN POLYMERSCHAUMSTOFF
METHOD FOR INCORPORATING ACTIVE AGENTS INTO A HYDROPHILIC POLYMER FOAM

(30) Priorité: 18.12.2012 FR 1262195
(43) Date de publication de la demande: 28.10.2015
(73) Titulaire: Urgo Recherche Innovation et Développement, 21300 Chenove (FR)
(72) Inventeur: PERNOT, Jean-Marc, Henri, Maurice, F-21000 Dijon (FR); RAVENET, Anne-Laure, F-71100 Chalon-sur-Saône (FR)
(74) Mandataire: Cabinet Beau de Loménie
(86) Numéro de dépôt international: PCT/FR2013/053111
(87) Numéro de publication internationale: WO 2014/096668

(56) Documents cités:
- WO-A2-2008/157711
- US-A- 5 098 621

## Description

La présente invention consiste en un nouveau procédé d'incorporation d'un ou plusieurs agents actifs hydrosolubles dans une mousse polymère hydrophile. Ledit procédé permet d'obtenir notamment une mousse polyuréthane hydrophile présentant un gradient d'agents actifs. Ce procédé est particulièrement utile à la fabrication notamment de mousses polyuréthane hydrophiles à destinées médicales, cosmétiques et dermo-cosmétiques.

### Etat de la technique antérieure

La plupart des mousses polymère classiques sont caractérisées par leur hydrophobicité.

Cependant, certains traitements nécessitent l'usage de mousses qui absorbent des exsudats, ainsi des mousses polymère hydrophiles ont été développées pour ces utilisations. Parmi les mousses polymère, on retrouve les mousses polyuréthane et les mousses d'alcool polyvinylique (PVA). Les mousses polyuréthane hydrophiles sont généralement préparées à partir d'un procédé dans lequel un prépolymère hydrophile possédant des groupements terminaux isocyanates est mélangé et mis en réaction avec de l'eau.

Dans les domaines médicaux, cosmétiques et dermo-cosmétiques, nombre de fabricants de mousses polymère hydrophiles ont par le passé proposé d'y incorporer des principes actifs d'intérêt efficaces notamment pour la cicatrisation de plaies.

On distingue essentiellement deux modes d'incorporation d'actifs dans les mousses polymère hydrophiles.

Une première méthode consiste en l'imprégnation d'une mousse par immersion de cette dernière dans une solution d'agent actif, cette étape étant suivi dans un second temps par une étape de séchage et d'évaporation du solvant. Ce type de procédé possède néanmoins un désavantage de taille : le fait d'exposer ce produit à des températures de séchage relativement élevées et pendant une période de temps relativement prolongée peut réduire l'activité du principe actif.

La seconde méthode d'incorporation d'un actif au sein d'une mousse polymère hydrophile particulièrement utilisée consiste en un mélange du principe actif avec les réactifs précurseurs de la mousse. Un problème commun à ces deux procédés d'incorporation d'actifs dans une mousse polymère hydrophile réside dans le dosage de l'agent actif à introduire. La fiabilité du dosage est d'autant plus sensible que les doses sont faibles. Ainsi, des pertes de composés peuvent être constatées. La plupart de ces principes actifs sont tout particulièrement onéreux, et la bonne gestion de leur dosage lors de leur incorporation dans le produit final est donc indispensable.

La demande de brevet US 5 098 621 de la société Twin Rivers a décrit un procédé de sprayage d'une couche de principe actif non hydrosoluble micro-empaqueté sur la surface intérieure du réservoir d'un applicateur. Une phase aqueuse comprenant eau, prépolymère et surfactant est ensuite coulée sur le support. Ce document propose un dispositif applicateur de principes actifs qui empêche le relargage prématuré du principe actif, par liaison de ce dernier à la mousse. Le principe actif piégé à la surface de la mousse est relargué uniquement sous l'action d'une contrainte mécanique. En effet, lors de l'étape de polymérisation de la mousse, les agents actifs microempactés hydrophobes restent intacts et se retrouvent ainsi accrochés à la surface de la mousse, garantissant leur intégrité, permettant ainsi leur libération uniquement après exercice d'une contrainte mécanique.

La demande de brevet WO 2006/007844 de la société Coloplast a proposé de réaliser un procédé d'adjonction d'actifs au sein d'un pansement absorbant résolvant les problèmes mis en exergue précédemment. La méthode de préparation décrite comprend le sprayage du principe actif sur la portion centrale de la surface de l'élément absorbant. Le principe actif se retrouve de façon préférée sur la surface ou dans les parties supérieures de l'épaisseur de l'élément absorbant. Par exemple, 90% du principe actif se trouve dans le quart supérieur de l'épaisseur de l'élément absorbant. Le sprayage dudit principe actif est réalisé au moyen d'un dispositif de sprayage LVLP (dispositif dispensant de faibles volumes de produits sous de faibles pressions). La couche absorbante de ce dispositif peut être préparée à partir des matériaux constitués parmi la liste des mousses, des alginates, des polysaccharides, des chitosans, des super-absorbants ou encore un mélange de ces derniers. Ce procédé de fabrication présente de nombreux inconvénients : il est réalisé en plusieurs étapes et l'incorporation par sprayage de l'agent actif au sein du dispositif absorbant est réalisée dans une étape ultérieure à celle de la fabrication dudit dispositif.

Enfin, la demande de brevet WO 2008/155711 de la société Rynel Inc. divulgue une méthode de préparation d'une mousse qui est imprégnée de particules de polymère hydrosoluble comprenant un agent actif.

Il apparaît donc nécessaire de réaliser un procédé plus avantageux économiquement, qui ne nécessite pas une étape de traitement supplémentaire postérieure à la réalisation du dispositif même, tout en garantissant un dosage précis de l'actif et un gradient d'actif dans l'épaisseur de la mousse.

La Demanderesse a mis au point un nouveau procédé répondant à ces problématiques, procédé d'incorporation d'un ou plusieurs agents actifs hydrosolubles dans une mousse polymère hydrophile comprenant les étapes de dépôt du ou des agents actifs hydrosolubles sur un support non poreux imperméable, préparation d'un mélange prépolymère en solution aqueuse, coulage dudit mélange obtenu sur ledit support, polymérisation et obtention d'une mousse polymère hydrophile incorporant un ou plusieurs actifs hydrosolubles, et enfin séchage de la mousse polymère hydrophile.

### Résumé de l'invention

Ainsi la présente invention consiste en un nouveau procédé d'incorporation d'au moins un agent actif hydrosoluble cosmétique ou pharmaceutique dans une mousse polymère hydrophile comprenant les étapes de :
a) Dépôt de l'agent actif hydrosoluble sur un support non poreux imperméable,
b) Préparation d'un mélange précurseur de la mousse sous forme d'une dispersion aqueuse contenant soit des monomères soit des prépolymères,
c) Coulage du mélange obtenu à l'étape b) sur l'agent actif qui a été déposé sur le support à l'issue de l'étape a),
d) Polymérisation et obtention de la mousse polymère hydrophile incorporant l'agent actif hydrosoluble,
e) Séchage de la mousse polymère hydrophile.

Dans ce procédé, l'agent actif se dissout dans la dispersion aqueuse contenant le mélange précurseur de la mousse, lors de son coulage sur le support à l'étape c) .

L'agent actif hydrosoluble est choisi parmi la liste constituée des agents antibactériens, des agents anti-inflammatoires, des agents kératolytiques, des agents immunomodulateurs, des agents chélateurs, des agents modifieurs de pH, des composés de la matrice extracellulaire, des agents favorisant la cicatrisation, des agents anti-viraux, des agents antifongiques et des agents anti-douleur.

L'agent actif hydrosoluble est de préférence de l'urée, de l'acide hyaluronique ou un oligosaccharide polysulfaté synthétique ayant 1 à 4 unités oses, ses sels ou complexes.

L'agent actif hydrosoluble peut être déposé seul sous forme pure ou en solution. Il peut également avoir été, préalablement à son dépôt, incorporé dans une matrice hydrosoluble.

Le support non poreux imperméable peut être par exemple un papier couché polyéthylène siliconé ou un film polyester siliconé.

Le prépolymère est notamment un prépolymère de type poly(alkylèneoxy)polyol.

On peut lui adjoindre un surfactant, notamment un surfactant de type cationique, anionique ou non ionique ou un surfactant à base de silicone.

### Description détaillée de l'invention

D'autres caractéristiques et avantages de l'invention apparaîtront plus clairement à la lecture de la description qui suit de deux modes de réalisation préférés de l'invention, donnés à titre d'exemples.

Ainsi la présente invention consiste en un nouveau procédé d'incorporation d'au moins un agent actif hydrosoluble cosmétique ou pharmaceutique dans une mousse polymère hydrophile comprenant les étapes de :
a) Dépôt de l'agent actif hydrosoluble sur un support non poreux imperméable,
b) Préparation d'un mélange précurseur de la mousse sous forme d'une dispersion aqueuse contenant soit des monomères soit des prépolymères,
c) Coulage du mélange obtenu à l'étape b) sur l'agent actif qui a été déposé sur le support à l'issue de l'étape a),
d) Polymérisation et obtention de la mousse polymère hydrophile incorporant l'agent actif hydrosoluble,
e) Séchage de la mousse polymère hydrophile,
caractérisé en ce que ledit agent actif se dissout dans la dispersion aqueuse contenant le mélange précurseur de la mousse, lors de son coulage sur le support à l'étape c), et en ce que ledit agent actif est choisi parmi la liste constituée des agents antibactériens, des agents anti-inflammatoires, des agents kératolytiques, des agents immunomodulateurs, des agents chélateurs, des agents modifieurs de pH, des composés de la matrice extracellulaire, des agents favorisant la cicatrisation, des agents anti-viraux, des agents antifongiques et des agents anti-douleur.

Préalablement à l'étape a), on peut préparer une solution ou une dispersion aqueuse de l'agent actif hydrosoluble que l'on dépose ensuite au cours de l'étape a) sur le support, par pulvérisation par exemple.

La mousse polymère peut être choisie parmi les mousses polyuréthane et les mousses d'alcool polyvinylique (PVA).

Selon un mode de réalisation préféré de l'invention, la mousse polymère est une mousse polyuréthane hydrophile.

Le prépolymère de la mousse polyuréthane hydrophile peut être déjà synthétisé.

Au contraire, si le prépolymère doit être préparé, il est possible de réaliser la mousse polyuréthane hydrophile en une ou deux étapes. En une étape, la mousse polyuréthane hydrophile est réalisée en mettant en réaction à la fois les polyols, les polyisocyanates, l'eau et un surfactant. En deux étapes, le prépolymère est synthétisé dans une étape préalable à l'adjonction d'eau et un surfactant, par mise en réaction des polyols et des polyisocyanates.

Si le prépolymère est déjà synthétisé, il suffit simplement de mettre en réaction ce dernier avec de l'eau et un surfactant pour obtenir une mousse polyuréthane hydrophile. Il est à noter que le surfactant ne constitue pas un composé essentiel quant à la réalisation d'une mousse polyuréthane hydrophile, il n'est qu'optionnel, mais joue un grand rôle sur le contrôle de la réaction et sur les caractéristiques physico-chimiques de la mousse obtenue.

Par « polyol », on entend au sens de la présente invention tout composé naturel ou synthétique présentant plusieurs groupements hydroxyles, lesdits groupements étant capables de réagir avec des groupements isocyanates pour aboutir à des ponts uréthane. Dans le cas de polyols d'origine synthétique, le polyéthylène glycol et les polyéthers sont les plus fréquemment utilisés par les fabricants de mousses polyuréthane hydrophiles notamment en raison de leur hydrophilie. Dans le cas de polyols d'origine naturelle, ceux-ci peuvent être dérivés des produits définis par la liste non limitative suivante : xylose, arabinose, glucose, saccharose, dextrines, glycérine, amidon, huile de ricin, huile de soja, huile végétale. Dans le cadre de la présente invention, les polyols préférentiellement utilisés seront du type poly(ethyleneoxy)polyol ou poly(propyleneoxy) polyols ou un mélange des deux.

Par « polyisocyanates », on entend tout composé pouvant être choisi parmi le groupe constitué du toluene-2,4-diisocyanate, du toluene-2,6-diisocyanate, de l'éthylidène diisocyanate, du propylene-1,2-diisocyanate, du cyclohexylene-1,2-diisocyanate, du cyclohexylene-1,4-diisocyanate, du triphenylmethane-4,4',4"-triisocyanate, du m-phénylène diisocyanate, du 4,4'-biphenylene diisocyanate, du 3,3'-dichloro-4,4'-biphenylene diisocyanate, du benzene-1,3,5-triisocyanate, du toluene-2,4,6-triisocyanate, diphenyl-2,4,4'-triisocyanate, du 1,4-tetraméthylène diisocyanate, du 1,6-hexaméthylène diisocyanate, du 1,10-decamethylene diisoycanate, du xylène diisocyanate, chlorophénylène diisocyanate, du diphenylmethane-4,4'diisocyanate, du naphthalene-1,5-diisocyanate, du cumene-2,4-diisocyanate, du 4-methoxy-1,3-phenylene diisocyanate, du 4-chloro-1,3-phenylene diisocyanate, du 4-bromo-1,3-phenylene diisocyanate, du 4-ethoxy-1,3-phenylene diisocyanate, du 4-bromo-1,3-phenylene diisocyanate, du 4-ethoxy-1,3-phenylene diisocyanate, du 2,4-diisocyanatodiphenyl éther, du 5,6-dimethyl-1,3-phenylene diisocyanate, du 2,4-dimethyl-1,3-phenylene diisocyanate, du 4,4'-diisocyanatodiphenyl éther, du benzidine diisocyanate, du xylène-alpha, de l'alpha diisothiocyanate, du 3,3'-dimethyl-4,4'-biphenylene diisocyanate, du 2,2',5,5'-tetramethyl-4,4'-biphenylene diisocyanate, du 4,4'-methylene-bis(phenylisocyanate), du 4,4'-sulfonylbis(phenylisocyanate), du 4,4'-methylene di-o-tolylisocyanate, de l'éthylène diisocyanate, de l'éthylène diisothiocyanate, et du triméthylène diisocyanate.

La mousse peut être une mousse polyuréthane hydrophile qui est obtenue par un mélange précurseur contenant un prépolymère de type poly(alkylèneoxy)-polyol.

Les prépolymères constitutifs d'une mousse polyuréthane hydrophile sont de préférence de type poly(alkylèneoxy)polyol. Parmi ces prépolymères, les prépolymères polyuréthane à groupements terminaux isocyanate sont particulièrement préférés comme par exemple les prépolymères commercialisés sous la marque Hypol® par Dow, Prepol® par Lendell Manufacturing Inc., Hydropol® par Mace Adhesives & Coatings Co., Aquapol® par Carpenter Co. et Urepol® par EnviroChem Technologies. Ces liaisons terminales isocyanate peuvent correspondre à des isocyanates aromatiques, comme par exemples le toluene diisocyanate (TDI) ou le méthylène diphényl isocyanate (MDI), ou encore de groupements isocyanates aliphatiques, comme par exemple l'isophorone diisocyanate (IPDI) mais aussi le méthylène diphenyl isocyanate hydrogéné (HMDI). Parmi les produits de la marque Hypol® pouvant être utilisés dans le cadre de la présente invention, on retrouve l'Hypol 2000®, l'Hypol 2002 E®, l'Hypol 3000®, l'Hypol 4000®, ou encore l'Hypol 5000®. Préférentiellement dans le cadre de la présente invention, le prépolymère utilisé dans la réalisation de la mousse polyuréthane hydrophile sera l'Hypol 2002 E®.

Le mélange précurseur de la mousse peut contenir un surfactant de type cationique, anionique ou non ionique ou un surfactant à base de silicone.

Selon un mode de réalisation, le mélange précurseur de la mousse contient un surfactant non ionique.

Une large variété de surfactants connue dans l'état de l'art peut être proposée quant à son incorporation dans la réalisation d'une mousse polyuréthane hydrophile. Parmi les surfactants connus, les surfactants cationiques, non ioniques ou encore les surfactants anioniques comme les sels d'acides gras, les sels d'esters d'acides sulfuriques, les sels d'esters d'acides phosphoriques et les sulfonates peuvent être présents dans la composition de base objet de la présente invention. Préférentiellement, les surfactants utilisés dans le cadre de la réalisation d'une mousse polyuréthane hydrophile seront des surfactants de type non ioniques tels que certains produits commercialisés par la société BASF sous le nom de Pluronic®. De manière encore plus préférée, le surfactant utilisé dans la réalisation d'une mousse polyuréthane hydrophile est le Pluronic PE6200®, copolymère d'oxyde d'éthylène et d'oxyde de propylène, ou encore le Pluronic PE6800®. Un autre type de surfactant connu utilisable dans le cadre de la présente invention est représenté par les surfactants à base de silicone. Parmi ceux-ci, on peut citer les copolymères hydrolysables de polysiloxane-polyoxyalkylène, les copolymères non hydrolysables de polysiloxane-polyoxyalkylène, les cyanoalkylpolysiloxanes, les alkylpolysiloxanes, les polydiméthylsiloxanes et les diméthylpolysiloxanes-polyoxyalkylène modifié. Le type de surfactant à base de silicone utilisé et la quantité nécessaire dépendent du type de mousse à produire.

Le procédé objet de la présente invention consiste en une incorporation d'un ou plusieurs agents actifs hydrosolubles au sein d'une mousse polyuréthane hydrophile classique. Cette incorporation se fait par dépôt du ou des agents actifs hydrosolubles sur un support non poreux et imperméable, dépôt préalable au coulage du mélange réactionnel précurseur de la mousse.

Par « support non poreux imperméable », on entend tout support destiné à être enduit par le mélange réactionnel donnant naissance à la mousse polyuréthane hydrophile. Préférentiellement, ce support est siliconé afin d'éviter que la mousse néoformée n'adhère sur ce dernier. A titre d'exemples, peut être utilisé en tant que support non poreux imperméable, un papier couché polyéthylène siliconé ou encore un film polyester siliconé.

Le support est de préférence séparé de la mousse après séchage de celle-ci, car il sert essentiellement de surface de coulage lors de la fabrication de la mousse, et n'est pas utile lors de l'utilisation de la mousse, notamment pour absorber les exsudats d'une plaie.

Aussi, selon un mode de réalisation particulier, le procédé de l'invention comprend une étape f) au cours de laquelle le support est séparé de la mousse après séchage de celle-ci à l'étape e).

Par « agent actif hydrosoluble », on entend au sens de la présente invention, tout composé actif se présentant sous la forme de particules solubles dans l'eau. Ainsi, des particules d'un agent actif hydrosoluble enrobées d'un matériau hydrophobe ne constituent pas un agent actif hydrosoluble au sens de l'invention.

Par « agent favorisant la cicatrisation », on entend au sens de la présente invention, tout composé choisi parmi la liste non limitative constituée du Rétinol, de la Vitamine A, de la Vitamine E et ses dérivés, de la N-Acétyl Hydroxyproline, des extraits de Centella *Asiatica,* de la Papaïne, de l'acide Hyaluronique, des oligosaccharides polysulfatés synthétiques ayant 1 à 4 unités d'oses, leurs sels et complexes, tels que par exemple les sels alcalins de sucrose octasulfate (parmi lesquels on retrouve entre autres, le sel de potassium de sucrose octasulfate ou encore le sel de sodium de sucrose octasulfate), le sucralfate, les sels d'acidés aminés de sucrose octasulfate, ou encore le sel d'argent de sucrose octasulfate, de l'allantoïne, et de la metformine.

Par « agent anti-viral », on entend au sens de la présente invention, tout composé choisi parmi la liste non limitative constituée de l'acyclovir, du famciclovir et du ritonavir.

Par « agent antifongique », on entend au sens de la présente invention, tout composé choisi parmi la liste non limitative constituée de la nystatine, de l'amphotéricine B, de la natamycine, du miconazole, du kétoconazole, du clotrimazole, de l'éconazole, du bifonazole, du butoconazole, du fenticonazole, de l'isoconazole, de l'oxiconazole, du sertaconazole, du sulconazole, du thiabendazole, du tioconazole, du fluconazole, de l'itraconazole, du ravuconazole, du posaconazole, du voriconazole, de l'allylamine, de la terbinafine, de l'amorolfine, de la naftifine, et du butenafine.

Par « agent anti-douleur », on entend au sens de la présente invention, tout composé choisi parmi la liste non limitative constituée du paracétamol, de la codéine, du dextropropoxyphène, du tramadol, de la morphine et ses dérivés et des corticoïdes et ses dérivés.

Par « agent anti-bactérien », on entend au sens de la présente invention, tout composé choisi parmi la liste non limitative constituée des sels ou complexes d'argent (tels les sulfates d'argent, les nitrates d'argent, les sulfamides d'argent ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, ou encore les probiotiques.

Par « agent anti-inflammatoire », on entend au sens de la présente invention, tout composé choisi parmi la liste non limitative constituée des anti-inflammatoires non stéroïdiens (AINS), de l'aspirine ou acide acétylsalicylique, de l'ibuprofène, du kétoprofène, du flurbiprofène, du diclofenac, l'acéclophénac, du kétorolac, du méloxicam, du piroxicam, du ténoxicam, du naproxène, de l'indométacine, du naproxcinod, le nimésulid, du célécoxib, de l'étoricoxib, le parécoxib, le rofécoxib, du valdécoxib, de la phénylbutazone, de l'acide niflumique, et de l'acide méfénamique.

Par « agent kératolytique », on entend au sens de la présente invention, tout composé choisi parmi la liste non limitative constituée de l'acide salicylique, du salicylate de zinc, de l'acide ascorbique, des acides alpha hydroxylés (tels que les acides glycolique, lactique, malique, citrique, tartrique), des extraits d'Erable argenté, de Griottier, ou de Tamarinier, de l'urée, de la Kératoline (commericalisée par la société Sederma), et de la papaïne.

Enfin, par « agent immunomodulateur », on entend le beta glucane, par agent chélateur, on entend l'acide éthylène diamine tétraacétique (EDTA) ainsi que les acides hydroxamiques, par agent modifieur de pH, on entend le bicarbonate de sodium, et par agent de la matrice extra-cellulaire, on entend, les glycosaminoglycanes, l'acide hyaluronique, le dermatane sulfate, l'héparane sulfate, les élastines, et la laminine.

De manière préférée, les agents actifs de l'invention sont l'urée (commercialisée par la société Fisher Scientific), l'acide hyaluronique (commercialisé par la société Soliance® sous le nom commercial Renovhyal®), ou encore un oligosaccharide polysulfaté synthétiques ayant 1 à 4 unités d'oses, ses sels et complexes, tels que par exemple les sels alcalins de sucrose octasulfate (parmi lesquels on retrouve entre autres, le sel de potassium de sucrose octasulfate ou encore le sel de sodium de sucrose octasulfate), le sucralfate, les sels d'acidés aminés de sucrose octasulfate, ou encore le sel d'argent de sucrose octasulfate

Lesdits agents actifs peuvent être incorporés dans la mousse en des quantités comprises entre 0.01% et 40% du poids final de la mousse polyuréthane hydrophile obtenue et préférentiellement entre 0.1 et 20% du poids final de la mousse polyuréthane hydrophile obtenue.

Ces agents actifs sont déposés sur le support non poreux imperméable sous forme de poudre, de solution, ou de dispersion aqueuse. Selon un autre mode de réalisation de l'invention, ils peuvent, préalablement, avoir été incorporés dans une matrice hydrosoluble.

Lorsque les agents actifs sont déposés sur le support non poreux et imperméable, il est possible de les déposer soit par saupoudrage sur la surface du support, soit de réaliser un simple sprayage d'un mélange eau/agent actif sur la surface du support en question. On choisit ou non dans ce dernier cas de sécher, par exemple par passage sous étuve, cet ensemble ainsi obtenu.

Les agents actifs peuvent être incorporés dans une matrice hydrosoluble avant d'être déposé sur le support. Aussi, préalablement à l'étape a), on peut préparer une matrice hydrosoluble comprenant l'agent actif hydrosoluble, matrice que l'on dépose ensuite au cours de l'étape a) sur le support.

Par « matrice hydrosoluble », on entend et ce quel que soit le type de matrice, tout matériau apte à se dissoudre dans l'eau sous une faible sollicitation mécanique pour donner une solution homogène, dans un intervalle de temps inférieur à 10 secondes et préférentiellement inférieur à 2 secondes à compter de la mise en contact dudit matériau dans ladite eau.

La matrice hydrosoluble possède un rôle de support de l'agent actif lors du coulage du mélange réactionnel permettant d'obtenir la mousse. Cette matrice disparaît dès sa mise en contact avec le mélange réactionnel constitutif de la mousse polyuréthane hydrophile, ou plutôt diffuse dans l'ensemble de ce mélange réactionnel, libérant ainsi les agents actifs hydrosolubles, agents actifs établissant dès lors un gradient au sein de la mousse formée. Les agents actifs sont donc libérés dans la mousse et diffusent dans la mousse selon un gradient de concentration perpendiculaire à la surface du support.

Dans le procédé de l'invention, la mousse polymère est hydrophile. Une matrice hydrophobe vise à maintenir l'agent actif en son sein pour qu'il soit libéré après le coulage du mélange réactionnel constitutif de la mousse Au contraire, la matrice hydrosoluble utilisée dans le cadre de l'invention permet de solubiliser l'agent actif au moment de la polymérisation de la mousse. L'utilisation d'une matrice hydrophobe est donc proscrite quel que soit le mode de réalisation envisagé de la présente invention.

Par « gradient d'actif », on entend tout agent actif présent de préférence dans les couches inférieures de la mousse, couches les plus proximales du support. De façon préférée 80% du poids total de l'agent actif introduit dans le procédé de fabrication de la mousse se trouve dans les 33% inférieurs de la mousse, et de façon encore plus préférée 90% du poids total de l'agent actif introduit dans le procédé de fabrication de la mousse se trouve dans les 33% inférieurs de la mousse.

L'avantage de disposer d'un tel gradient d'agent actif réside dans l'offre d'une meilleure disponibilité de l'agent actif à la surface de la plaie ou de la peau de l'individu.

La détermination des valeurs de gradient d'agents actifs, au sein d'une mousse polyuréthane hydrophile est effectuée par l'intermédiaire d'une méthode classique de mesure du relargage du ou des agents actifs contenu dans chaque tranche de mousse testée. Les valeurs de relargage d'agent actif ainsi obtenues sont proportionnelles aux quantités d'agent actif contenues dans chaque tranche de mousse testée et permettent d'obtenir la valeur de gradient d'agent actif dans la mousse.

Les agents actifs peuvent être incorporés dans une matrice hydrosoluble sous la forme soit d'agglomérats soit d'un voile de nanofibres.

Par « agglomérat », on entend toute agrégation de particules liées entre elles, lesdites particules étant constituées pour 60 % au moins, et de préférence 80 % au moins d'entre elles d'un (ou plusieurs) matériau(x) choisi(s) parmi les polysaccharides, les protéines et des polymères synthétiques, et apte à se solubiliser en moins de 10 secondes et de préférence encore en moins de 2 secondes dès sa mise en contact dans de l'eau. Ces agglomérats contiennent des agents actifs hydrosolubles tels que définis *supra.* Selon un mode de réalisation particulier de l'invention, les agglomérats contiennent un (ou plusieurs) agent(s) actif(s). Dans le cas où une pluralité d'agents actifs est utilisée, ceux-ci pourront être incorporées soit individuellement dans des agglomérats séparés, soit sous forme de mélange au sein d'un même agglomérat. Un agglomérat peut notamment désigner un ensemble de particules de petites tailles, c'est à dire ayant une dimension moyenne comprise entre 500 nm et 1000 µm, de préférence comprise entre 1 µm et 500 µm, liées entre elles, par exemple en forme de framboise.

Chaque agglomérat a généralement une dimension moyenne comprise entre 5 et 2 000 µm et sera donc constitué d'environ 10 à 10 000 particules, de préférence d'environ 100 à 1000 particules.

De tels agglomérats de particules peuvent être préparés, de façon connue en soi par des procédés de granulation en voie humide comme en particulier par spray-drying ou dans un lit d'air fluidisé en utilisant généralement l'eau ou un mélange aqueux comme liant.

La quantité d'agglomérats déposée peut varier dans de larges proportions et sera généralement comprise entre 1 et 500 grammes par mètre carré, de préférence comprise entre 5 et 100 g/m².

Parmi les polysaccharides susceptibles d'être utilisés dans la réalisation de tels agglomérats, on peut citer l'amidon, les amidons modifiés, la maltodextrine, les gommes comme en particulier la gomme arabique ou la gomme d'acacia, la cellulose, les dérivés cellulosiques comme en particulier la méthylcellulose, la carboxyméthylcellulose, l'hydroxypropylcellulose, l'hydroxyéthylcellulose, les sucres, la pectine et les alginates.

Parmi les protéines susceptibles d'être utilisées dans la réalisation de tels agglomérats, on peut citer la gélatine, l'albumine et la caséine.

Parmi les polymères synthétiques susceptibles d'être utilisés dans la réalisation de tels agglomérats, on peut citer les polyacrylates ou encore l'alcool polyvinylique.

Par « voile de nanofibres », on entend tout ensemble de nanofibres ayant un diamètre compris entre 20 et 1 000 nm, et de préférence encore compris entre 50 et 500 nm disposées , de préférence selon une disposition monocouche, en formant un voile analogue à un non-tissé, c'est à dire un assemblage aléatoire de nanofibres maintenu sous forme d'une nappe par friction, cohésion ou adhésion.

Ce voile de nanofibres constituées pour 60% au moins, et de préférence 80 % au moins d'entre elles d'un (ou plusieurs) matériau (x) choisi(s) parmi les polymères naturels ou synthétiques, et apte à se solubiliser en moins de 10 secondes et de préférence encore en moins de 2 secondes dès sa mise en contact dans de l'eau.

Les voiles de nanofibres selon l'invention peuvent être obtenus par étirage aérodynamique de polymères fondus ou en solution aqueuse ou solvant selon la technologie connue sous le nom de "centrifugal spinning". Les voiles de nanofibres peuvent également être obtenus par le procédé connu sous le nom d' "électrofilage" ou "électrospinning".

L'électrofilage est une technologie permettant la réalisation de nanofibres par évaporation d'une solution ou d'une dispersion de polymère au sein d'un champ électrique à potentiel élevé. Plus précisément, ce procédé consiste à soumettre une solution ou dispersion d'un matériau suffisamment fluide, sortant d'une buse très fine, à un potentiel électrique de l'ordre de 5 à 50 kV. Sous l'effet de ce champ électrique, la goutte sortant de la buse se charge électriquement en prenant une forme sensiblement conique et peut être étirée en jet pour former une fibre très fine de taille nanométrique à micrométrique lorsque la tension électrique est suffisamment élevée pour rompre la tension superficielle de la goutte. Les fibres ainsi formées peuvent être recueillies et conservées en l'état par exemple sur un collecteur, ou bien encore déposées sur un support de façon aléatoire pour former un voile semblable à un non tissé. Cette technologie est particulièrement adaptée à la préparation de microfibres ou nanofibres à base d'alcool polyvinylique.

Parmi les matériaux susceptibles d'être utilisés selon l'invention, on peut citer l'alcool polyvinylique (PVA); la polyvinyl-pyrrolidone (PVP); le polyéthylènimine (PEI); les oxydes de polyéthylène (PEO); la carboxyméthylcellulose; les alginates; et les mélanges de ces composés.

Dans le cas où le voile de nanofibres est préparé par électrofilage, les agents actifs pourront être incorporés dans la solution ou dispersion soumise au champ électrique, permettant ainsi d'intégrer ces agents actifs directement dans le voile de nanofibres. Lesdits agents actifs peuvent aussi être éventuellement déposés sur le réceptacle des nanofibres.

La quantité de nanofibres déposée peut varier dans de larges proportions et sera généralement comprise entre 0.5 et 200 g, et de préférence entre 1 et 30 g par mètre carré de masse adhésive.

### Exemples

### Exemple 1 : Réalisation d'une mousse polyuréthane hydrophile incorporant du sucrose octasulfate de potassium :

Au fond d'un bécher jetable, on dépose un papier couché polyéthylène siliconé sur lequel on a sprayé une solution à une teneur de 5% en sucrose octasulfate de potassium.

Dans une seconde étape, on ajoute dans un second bécher 2 grammes de Pluronic PE6200® à de l"eau distillée d'un poids de 98 grammes. On homogénéise cette solution à l'aide d'un mélangeur à hélice à 500 tours/min.

Dans une troisième étape on reprend 40 grammes de la solution finale réalisée à l'étape 2, à laquelle on ajoute 20 grammes d'Hypol 2002®. On homogénéise ce mélange pendant une durée de 20 secondes à l'aide d'un mélangeur à hélice à 800 tours/min.

Enfin dans une quatrième et dernière étape, on coule le mélange réactionnel obtenu à la fin de l'étape 3 dans le bécher contenant le support sur lequel a été sprayé préalablement la solution d'une teneur de 5% en sucrose octasulfate de potassium, correspondant au support obtenu à la fin de l'étape 1. On laisse gonfler la mousse puis on la laisse sécher sous étuve à 70°C pendant au moins 4 heures.

On obtient ainsi après séparation de la mousse obtenue du support, une mousse polyuréthane hydrophile incorporant du sucrose octasulfate de potassium.

### Exemple 2 : Réalisation d'une mousse polyuréthane hydrophile incorporant de l'urée :

Au fond d'un bécher jetable, on répartit de façon régulière sur du papier couché polyéthylène siliconé 2 grammes d'urée sous forme de poudre.

Dans une seconde étape, on ajoute dans un second bécher 2 grammes de Pluronic PE6200® à de l'eau distillée d'un poids de 98 grammes. On homogénéise cette solution à l'aide d'un mélangeur à hélice à 500 tours/min.

Dans une troisième étape on reprend 40 grammes de la solution finale réalisée à l'étape 2, à laquelle on ajoute 20 grammes d'Hypol 2002®. On homogénéise ce mélange pendant une durée de 20 secondes à l'aide d'un mélangeur à hélice à 800 tours/min.

Enfin dans une quatrième et dernière étape, on coule le mélange réactionnel obtenu à la fin de l'étape 3 sur le support sur lequel est répartit de façon régulière une quantité de 2 grammes d'urée sous forme de poudre, correspondant au support obtenu à la fin de l'étape 1. On laisse gonfler la mousse puis on la laisse sécher sous étuve à 70°C pendant au moins 4 heures.

On obtient ainsi après séparation de la mousse obtenue du support, une mousse polyuréthane hydrophile incorporant de l'urée.

### Exemple 3 : Détermination des valeurs de gradient d'agents actifs de la mousse polyuréthane hydrophile réalisée selon l'exemple 1 :

La détermination des valeurs de gradient d'agents actifs, à savoir de sucrose octasulfate de potassium, au sein de la mousse polyuréthane hydrophile réalisée selon l'exemple 1, est effectuée par l'intermédiaire d'une méthode de mesure du relargage du ou des agents actifs contenu dans la mousse, ou la tranche de mousse en question.

La quantité d'agent actif relarguée est proportionnelle à la quantité d'agent actif présente dans la tranche de mousse en question.

Dans le présent cas, une tranche de mousse de 3 mm d'épaisseur et d'environ 25 cm² est découpée à partir du tiers inférieur, médian et supérieur de la mousse néoformée. Chaque tranche est représentative de chaque tiers de mousse. Chaque tranche est immergée dans du sérum physiologique pendant 24 heures à 37°C. Le sérum physiologique est récupéré et dosé par HPLC. Les valeurs de relargage sont ainsi connues.

On obtient donc :

| Test | Unités | Echantillons analysés | Relargage moyen |
|---|---|---|---|
| Relargage d'agent actif contenu dans la mousse réalisée selon l'exemple 1 | % | Inférieur | 99.8 |
| | | Médian | 0.1 |
| | | Supérieur | 0.1 |

Le relargage d'actif de chaque tranche est exprimé en fonction de la quantité d'actif de la solution sur laquelle le mélange réactionnel est coulé.

Ainsi la quasi-totalité de la quantité d'agent actif (99,8%) se retrouve dans la partie basse de la mousse, partie proximale de la peau de l'individu.

Ainsi 99,8% de l'agent actif se retrouve dans le tiers inférieur de la mousse.

## Revendications

1. Procédé d'incorporation d'au moins un agent actif hydrosoluble cosmétique ou pharmaceutique dans une mousse polymère hydrophile comprenant les étapes de :
a) Dépôt de l'agent actif hydrosoluble sur un support non poreux imperméable,
b) Préparation d'un mélange précurseur de la mousse sous forme d'une dispersion aqueuse contenant soit des monomères soit des prépolymères,
c) Coulage du mélange obtenu à l'étape b) sur l'agent actif qui a été déposé sur le support à l'issue de l'étape a),
d) Polymérisation et obtention de la mousse polymère hydrophile incorporant l'agent actif hydrosoluble,
e) Séchage de la mousse polymère hydrophile,
**caractérisé en ce que** ledit agent actif se dissout dans la dispersion aqueuse contenant le mélange précurseur de la mousse, lors de son coulage sur le support à l'étape c), et **en ce que** ledit agent actif est choisi parmi la liste constituée des agents antibactériens, des agents anti-inflammatoires, des agents kératolytiques, des agents immunomodulateurs, des agents chélateurs, des agents modifieurs de pH, des composés de la matrice extracellulaire, des agents favorisant la cicatrisation, des agents anti-viraux, des agents antifongiques et des agents anti-douleur.

2. Procédé selon la revendication 1, **caractérisé en ce que** la mousse est une mousse polyuréthane hydrophile qui est obtenue par un mélange précurseur contenant un prépolymère de type poly(alkylèneoxy)polyol.

3. Procédé selon les revendications 1 ou 2, **caractérisé en ce que** le mélange précurseur de la mousse contient un surfactant à base de silicone ou un surfactant de type cationique, anionique ou non ionique.

4. Procédé selon l'une quelconque des revendications précédentes, **caractérisé en ce que** le mélange précurseur de la mousse contient un surfactant non ionique.

5. Procédé selon l'une quelconque des revendications précédentes, **caractérisé en ce que** l'agent actif hydrosoluble est l'urée, l'acide hyaluronique ou un oligosaccharide polysulfaté synthétique ayant 1 à 4 unités d'oses, ses sels et complexes.

6. Procédé selon l'une quelconque des revendications précédentes, **caractérisé en ce que**, préalablement à l'étape a), on prépare une solution ou une dispersion aqueuse de l'agent actif hydrosoluble que l'on dépose ensuite au cours de l'étape a) sur le support, par pulvérisation par exemple.

7. Procédé selon l'une quelconque des revendications 1 à 5, **caractérisé en ce que**, préalablement à l'étape a), on prépare une matrice hydrosoluble comprenant l'agent actif hydrosoluble, matrice que l'on dépose ensuite au cours de l'étape a) sur le support.

8. Procédé selon l'une quelconque des revendications précédentes, **caractérisé en ce que** le support non poreux imperméable est un papier couché polyéthylène siliconé ou un film polyester siliconé.

9. Procédé selon l'une quelconque des revendications précédentes, **caractérisé en ce qu'**il comprend une étape f) au cours de laquelle le support est séparé de la mousse après séchage de celle-ci à l'étape e).

## Patentansprüche

1. Verfahren zur Einbeziehung zumindest eines wasserlöslichen kosmetischen oder pharmazeutischen Wirkstoffs in einen hydrophilen Polymerschaum, umfassend die Schritte:
a) Abscheiden des wasserlöslichen Wirkstoffs auf einem nicht porösen, undurchlässigen Träger,
b) Herstellen einer Vorläufermischung des Schaums in Form einer wässrigen Dispersion, die entweder Monomere oder Vorpolymere enthält,
c) Gießen der in Schritt b) erhaltenen Mischung auf den Wirkstoff, der am Ende von Schritt a) auf dem Träger abgeschieden wurde,
d) Polymerisation und Erhalten des hydrophilen Polymerschaums, der den wasserlöslichen Wirkstoff einbezieht,
e) Trocknen des hydrophilen Polymerschaums,
**dadurch gekennzeichnet, dass** der Wirkstoff sich in der wässrigen Dispersion, welche die Vorläufermischung des Schaums enthält, bei deren Gießen auf den Träger in Schritt c) auflöst, und dass der Wirkstoff aus der Liste ausgewählt ist, die durch antibakterielle Mittel, entzündungshemmende Mittel, keratolytische Mittel, immunmodulierende Mittel, chelatbildende Mittel, pH-modifizierende Mittel, Verbindungen der extrazellulären Matrix, vernarbungsfördernde Mittel, antivirale Mittel, antimykotische Mittel und Schmerzmittel gebildet wird.

2. Verfahren nach Anspruch 1, **dadurch gekennzeichnet, dass** der Schaum ein hydrophiler Polyurethanschaum ist, der durch eine Vorläufermischung erhalten wird, die ein Vorpolymer vom Typ Poly(alkylenoxy)polyol enthält.

3. Verfahren nach den Ansprüchen 1 oder 2, **dadurch gekennzeichnet, dass** die Vorläufermischung des Schaums einen oberflächenaktiven Stoff auf Basis von Silikon oder einen oberflächenaktiven Stoff vom kationischen, anionischen oder nicht ionischen Typ enthält.

4. Verfahren nach einem der vorhergehenden Ansprüche, **dadurch gekennzeichnet, dass** die Vorläufermischung des Schaums einen nicht ionischen oberflächenaktiven Stoff enthält.

5. Verfahren nach einem der vorhergehenden Ansprüche, **dadurch gekennzeichnet, dass** der wasserlösliche Wirkstoff aus Harnstoff, Hyaluronsäure oder einem synthetischen polysulfatierten Oligosaccharid, das 1 bis 4 Monosaccharideinheiten aufweist, dessen Salzen und Komplexen besteht.

6. Verfahren nach einem der vorhergehenden Ansprüche, **dadurch gekennzeichnet, dass** vor dem Schritt a) eine Lösung oder eine wässrige Dispersion des wasserlöslichen Wirkstoffs hergestellt wird, die in der Folge im Verlauf von Schritt a) auf dem Träger abgeschieden wird, zum Beispiel durch Zerstäuben.

7. Verfahren nach einem der Ansprüche 1 bis 5, **dadurch gekennzeichnet, dass** vor dem Schritt a) eine wasserlösliche Matrix umfassend den wasserlöslichen Wirkstoff hergestellt wird, wobei die Matrix in der Folge im Verlauf von Schritt a) auf dem Träger abgeschieden wird.

8. Verfahren nach einem der vorhergehenden Ansprüche, **dadurch gekennzeichnet, dass** der nicht poröse, undurchlässige Träger ein silikonisiertes, mit Polyethylen beschichtetes Papier oder eine silikonisierte Polyesterfolie ist.

9. Verfahren nach einem der vorhergehenden Ansprüche, **dadurch gekennzeichnet, dass** es einen Schritt f) umfasst, in dessen Verlauf der Träger von dem Schaum nach dessen Trocknung in Schritt e) getrennt wird.

## Claims

1. A process for the incorporation of at least one cosmetic or pharmaceutical water-soluble active agent in a hydrophilic polymer foam, comprising the stages of:
a) deposition of the water-soluble active agent on an impermeable nonporous support,
b) preparation of a precursor mixture of the foam in the form of an aqueous dispersion comprising either monomers or prepolymers,
c) pouring the mixture obtained in stage b) over the active agent which has been deposited on the support at the end of stage a),
d) polymerization and production of the hydrophilic polymer foam incorporating the water-soluble active agent,
e) drying the hydrophilic polymer foam.
**characterized in that** said active agent dissolves in the aqueous dispersion comprising the precursor mixture of the foam during the pouring of stage c), and **in that** said active agent is chosen from the list consisting of antibacterial agents, antiinflammatory agents, keratolytic agents, immunomodulating agents, chelating agents, pH-modifying agents, compounds of the extracellular matrix, agents which promote healing, antiviral agents, antifungal agents and painkillers.

2. The process as claimed in claim 1, **characterized in that** the foam is a hydrophilic polyurethane foam which is obtained with a precursor mixture comprising a prepolymer of poly(alkyleneoxy) polyol type.

3. The process as claimed in claim 1 or claim 2, **characterized in that** the precursor mixture of the foam comprises a silicone-based surfactant or a surfactant of cationic, anionic or nonionic type.

4. The process as claimed in any one of the preceding claims, **characterized in that** the precursor mixture of the foam comprises a nonionic surfactant.

5. The process as claimed in any one of the preceding claims, **characterized in that** the water-soluble active agent is urea, hyaluronic acid or a synthetic polysulfated oligosaccharide having from 1 to 4 monosaccharide units, its salts and its complexes.

6. The process as claimed in any one of the preceding claims, **characterized in that**, prior to stage a), an aqueous solution or dispersion of the water-soluble active agent is prepared, which is subsequently deposited on the support during stage a), for example by spraying.

7. The process as claimed in any one of claims 1 to 5, **characterized in that**, prior to stage a), a water-soluble matrix comprising the water-soluble active agent is prepared, which matrix is subsequently deposited on the support during stage a) .

8. The process as claimed in any one of the preceding claims, **characterized in that** the impermeable nonporous support is a silicone-treated polyethylene-coated paper or a silicone-treated polyester film.

9. The process as claimed in any one of the preceding claims, **characterized in that** it comprises a stage f) during which the support is separated from the foam after the latter has been dried in stage e).
